# EUROPEAN PATENT APPLICATION

(11) **EP 2 314 287 A1**
(43) Date of publication of application: **27.04.2011**
(21) Application number: 09803037.2
(22) Date of filing: 31.07.2009
(51) Int. Cl.: A61K 9/70, A61L 15/58

(54) **PRESSURE-SENSITIVE ADHESIVE SHEET TO BE STUCK TO THE SKIN**

(30) Priority: 31.07.2008 JP 2008198801
(71) Applicant: Kaneka Corporation, Osaka-shi, Osaka 530-8288 (JP)
(72) Inventor: TANIGUCHI, Shuhei, Settsu-shi Osaka 566-0072 (JP); GOTO, Masaoki, Settsu-shi Osaka 566-0072 (JP); UEDA, Kazuhiko, Osaka-shi Osaka 530-8288 (JP)
(74) Representative: TBK-Patent
(86) International application number: PCT/JP2009/063634
(87) International publication number: WO 2010/013799

(57) **Abstract**

A pressure-sensitive adhesive sheet to be stuck to the skin which comprises a support and a pressure-sensitive adhesive layer located on the support, **characterized in that** the pressure-sensitive adhesive layer is formed by curing a composition containing (A) a polyoxyalkylene polymer having at least one alkenyl group per molecule, (B) a compound having hydrosilyl groups at both ends, (C) a compound having two or more hydrosilyl groups on average per molecule, and (D) a hydrosilylation catalyst. Compared with the existing pressure-sensitive adhesive sheets to be stuck to the skin, the pressure-sensitive adhesive sheet to be stuck to the skin as described above little irritates the skin and can achieve a sufficient pressure-sensitive adhesion force and a long-lasting fixation force to the skin.

## Description

### Technical Field

The present invention relates to a pressure-sensitive adhesive sheet to be stuck to the skin for use in the medical field.

### Background Art

Adhesive sheets in various shapes, such as surgical tapes, adhesive bandages, skin-protecting materials, film dressings and hydrocolloid dressings for treatment of wounds, stoma-care adhesive materials, percutaneous absorbing materials, and base materials for measurement of electrocardiogram, have been used in the medical field. Rubber-based adhesives have been used for adhesive sheets for use as adhered to the skin, but the rubber-based adhesives generally have low moisture permeability, often causing problems such as rash. For that reason, acrylic adhesives superior in adhesiveness and moisture permeability are used (see, for example, Patent Document 1). However, such an acrylic polymer is generally coated, for example, on a support base material or a liner (peel-away backing), after the viscosity of the solution thereof is adjusted, for example, by using an organic solvent. The organic solvent or the like used for viscosity adjustment is removed by vaporization after application, but because completely removal thereof is difficult, the organic solvent remaining in the adhesive may be absorbed percutaneously into the body, causing inflammation such as rash.

Silicone-based adhesives are proposed as the adhesives that can reduce such physical irritation to the skin and do not contain any organic solvent (see, for example, Patent Document 2). It is possible with such an adhesive to reduce irritation to the skin, because its moisture permeability is high, and to eliminate the potential skin-irritating factor, because no organic solvent is used therein. However, the silicone-based adhesives may not have sufficient adhesive strength and may not be suitable for adhesion to the skin for a long period.

### Citation List

### Patent Literature

Patent Document 1 JP No. 3190713
Patent Document 2 JP-A No. 2005-110875

### Summary of Invention

### Technical Problem

An object of the present invention is to provide a pressure-sensitive adhesive sheet to be stuck to the skin that is less irritating to the skin, has sufficient adhesive force and can be adhered to the skin for an extended period of time, compared to traditional pressure-sensitive adhesive sheets to be stuck to the skin.

### Solution to Problem

After intensive studies to solve the problems above, the inventors made the present invention. Specifically, the present invention relates to a pressure-sensitive adhesive sheet to be stuck to the skin having a support and a pressure-sensitive adhesive layer formed thereon, characterized in that, the pressure-sensitive adhesive layer is prepared by curing an adhesive agent composition containing (A) a polyoxyalkylene polymer having at least one alkenyl group in one molecule, (B) a compound having hydrosilyl groups at both ends, (C) a compound having two or more hydrosilyl groups on average in one molecule, and (D) a hydrosilylation catalyst.

In a favorable embodiment of the present invention, the compound (B) is a compound represented by the following Formula (1).

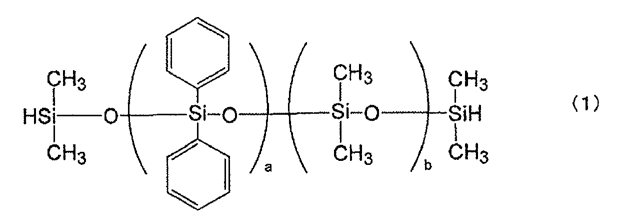

In another favorable embodiment of the present invention, the compound (B) has a number-average molecular weight of 300 to 4000.

In another favorable embodiment of the present invention, the compound (B) has a refractive index of 1.45 to 1.50 at 25°C.

In yet another favorable embodiment of the present invention, the compound (B) has a viscosity of 5 to 500 cP at 25°C.

In yet another embodiment of the present invention, the polyoxyalkylene polymer (A) has a number-average molecular weight of 3000 to 50000.

In yet another embodiment of the present invention, the main chain of the polyoxyalkylene polymer (A) is polyoxypropylene.

In yet another embodiment of the present invention, the ratio of [total amount of hydrosilyl group in component (B)]/[total amount of alkenyl group in component (A)] is 0.1 to 0.9 in the adhesive agent composition.

### Advantageous Effects of Invention

It is possible to provide a pressure-sensitive adhesive sheet to be stuck to the skin that is less irritating to the skin, has sufficient adhesive force and can be adhered to the skin for an extended period of time, compared to traditional pressure-sensitive adhesive sheets to be stuck to the skin.

### Description of Embodiments

Hereinafter, the present invention will be described in detail.

The pressure-sensitive adhesive sheet to be stuck to the skin according to the present invention has a support and at least one pressure-sensitive adhesive layer formed on the substrate.

The material for the support is not particularly limited, and those used normally for skin patches can be used.

Typical examples thereof include films of urethane-based polymers such as polyether urethane; amide-based polymers such as polyether amide; acrylic polymers such as polyacrylate; olefinic polymers such as polyethylene, polypropylene and ethylene-vinyl acetate copolymers; ester-based polymers such as polyether polyester; fluorochemical polymers such as polytetrafluoroethylene; silicone-based polymers such as polydimethylsiloxane and polydiphenylsiloxane; film of polyvinylchloride, polyvinylidene chloride, polyvinylalcohol and the like; foamed sheets of the above-mentioned polymeric materials; nonwoven fabrics; metal foil and the like. These materials may be used alone or in combination of two or more, and a moisture-permeable polyurethane film or a nonwoven fabric is used favorably.

The thickness of the support is preferably in the range of 5 to 2000 µm, more preferably 5 to 1000 µm, from the points of skin irritation and durability A support's thickness of more than 2000 µm often unfavorably leads to easier separation thereof because of deterioration in compatibility with the skin and increase of the physical irritation to the skin. A support's thickness of less than 5 µm is not favorable from the viewpoint of durability.

The pressure-sensitive adhesive layer is a pressure-sensitive adhesive layer prepared by curing a composition containing (A) a polyoxyalkylene polymer having at least one alkenyl group in one molecule, (B) a compound having hydrosilyl groups at both ends, (C) a compound having two or more hydrosilyl group on average in one molecule, and (D) a hydrosilylation catalyst. The term "curing" means hydrosilylation reaction that proceeds among the polymer (A) and the compounds (B) and (C) under heat. An example of the curing condition is 40 to 180°C for 1 to 60 minutes. The pressure-sensitive adhesive layer may be left at 40 to 80°C additionally for several days for more complete curing.

The polymer (A) is a polyoxyalkylene polymer having at least one alkenyl group in one molecule. The alkenyl group is not particularly limited, if it is a compound having a carbon-carbon double bond reactive in the hydrosilylation reaction. Examples of the alkenyl groups include aliphatic unsaturated hydrocarbon groups preferably having 2 to 20 carbon atoms, more preferably 2 to 6 carbon atoms (such as vinyl, allyl, methylvinyl, propenyl, butenyl, pentenyl, hexenyl, etc.), cyclic unsaturated hydrocarbon groups preferably having 3 to 20 carbon atoms, more preferably 3 to 6 carbon atoms (such as cyclopropenyl, cyclobutenyl, cyclopentenyl, cyclohexenyl, etc.), a methacryl group and the like.

The alkenyl group is preferably a group represented by the following Formula (2) or (3) for easier hydrosilylation reaction with the compounds (B) and (C). In the following Formulae, R¹ or R² is a hydrogen atom or a hydrocarbon group having 1 to 10 carbon atoms, and preferably it is a hydrogen atom or a methyl group.

(2) H₂C=C(R¹)-

(3) HC(R²)=CH-

The polymer (A) has averagely at least one, preferably 1 to 5, more preferably 1 to 3, more preferably 1 to 2 alkenyl groups in one molecule. When the number of the alkenyl group per mole of the polymer (A) is less than 1, curing efficiency is insufficient, while, when the number of the alkenyl group in the molecule is too large, the crosslinked structure becomes denser, often leading to deterioration of adhesive properties.

The polyoxyalkylene polymer of the polymer (A) is not particularly limited, and various polymers can be used. Typical examples thereof include polymers having a recurring unit represented by General Formula (-R³-O-) as the main chain. In the Formula, -R³- represents a bivalent alkylene group. The polyoxyalkylene polymer may have one kind of recurring unit or multiple kinds of recurring units. The polyoxyalkylene polymer may be a linear polymer or a branched polymer.

The main chain of the polymer (A) is favorably polyoxypropylene (i.e., -R³- above is -CH₂ CH(CH₃)-), from the points of adhesive properties, skin irritation, and skin wettability. Polymers (A) having polyoxypropylene as the main chain are favorable from the points of commercial availability and processability.

All of the regions of the polymer (A) other than those of alkenyl groups preferably have polyoxyalkylene skeletons, but the region may contain other structural units too. In such a case, the total amount of the polyether skeleton in the polymer (A) is preferably 80 wt % or more, more preferably 90 wt % or more.

The number-average molecular weight of the polymer (A) is preferably 3000 to 50000, more preferably 5000 to 50000, particularly preferably 5000 to 40000, from the points of processability at room temperature and adhesive properties. A polymer (A) having a number-average molecular weight of less than 3000 often gives a cured product that is brittler, while a polymer (A) having a number-average molecular weight of more than 50000 is more viscous, leading to deterioration in processability. The molecular weight above is the number-average molecular weight as polystyrene that is determined by GPC. The bond of the alkenyl group to the polyether-based polymer is not particularly limited, and examples thereof include direct bond of alkenyl group ether, ester bond, carbonate bond, urethane bond, urea bond, and the like.

The production method of the polymer (A) is not particularly limited, and the polymer is produced, for example, by a method of preparing a polyether-based polymer and then introducing an alkenyl group. In this case, the polyether-based polymer can be prepared by various known production methods, and a commercially available polyether-based polymer may also be used.

Various methods are usable as the methods of introducing an alkenyl group to the polyoxyalkylene polymer. It can be accomplished, for example, by copolymerization of an alkenyl group-containing monomer such as allyl glycidyl ether with an oxyalkylene monomer. It is also possible to introduce an alkenyl group to the main or side, by reacting an oxyalkylene polymer having a functional group such as hydroxyl or alkoxide in the main or side chain with an organic compound having a functional group reactive to one of these functional groups and an alkenyl group. In particular, presence of the alkenyl groups at the terminals of the main chain is favorable, because the effective crosslinking chain length is elongated in the cured product, and thus, a cured product superior in mechanical properties can be obtained.

Examples of the compounds having a functional group reactive with the functional group and an alkenyl group include acrylic acid, methacrylic acid, vinyl acetate, acid halides of unsaturated fatty acids having 3 to 20 carbon atoms such as acrylic chloride or acrylic bromide, acid anhydrides, allyl chloroformate, allyl chloride , allyl bromide, vinyl(chloromethyl)benzene, allyl(chloromethyl)benzene, allyl(bromomethyl)benzene, allyl(chloromethyl)ether, 1-hexenyl(chloromethoxy)benzene, allyloxy(chloromethyl)benzene, and the like.

The compound (B) is a compound having hydrosilyl groups at both ends. The hydrosilyl group means a group having a Si-H bond. In the present invention, if two hydrogen atoms (H) are bound to the same silicon atom (Si), the compound has two hydrosilyl groups. More typical examples of the compounds (B) are represented by the following Formula (4).

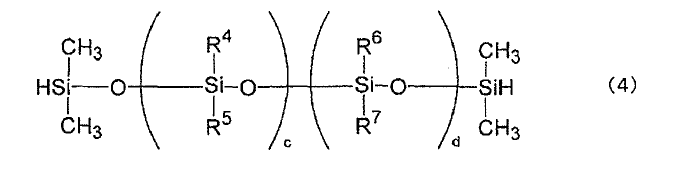

In Formula (4) above, c and d independently represent 0 (zero) or a positive integer. The values of c and d are not particularly limited, if both of c and d are not zero at the same time. The value of c+d is also preferably 2 to 50, although it is not particularly limited thereto.

R⁴ to R⁷ each represent a hydrocarbon group having 1 to 20 carbon atoms in the main chain. Hydrocarbon groups favorable from the points of compatibility with polymer (A), compound (C), and catalyst (D) or of dispersion stability are methyl, ethyl, propyl, isopropyl, phenyl, and the like.

The number-average molecular weight of the compound (B) is preferably 300 to 4000, more preferably 300 to 3000, from the point of processability at room temperature. A compound having a number-average molecular weight of less than 300 vaporizes during curing under heat, prohibiting production of a sufficient cured product, while a compound having a molecular weight of more than 4000 prohibits sufficiently high curing speed.

The refractive index of the compound (B) is preferably in the range of 1.45 to 1.55 at 25°C, more preferably in the range of 1.45 to 1.50 and particularly preferably 1.47 to 1.49. A compound (B) having a refractive index in the range above is preferable, as it is superior in compatibility with the polymer (A). When the refractive index of the compound (B) is not in the range above, the compound (B) is less compatible with the polymer (A), leading to insufficient reaction with the polymer (A) by phase separation and thus, possibly prohibiting production of an adhesive layer having satisfactory adhesive properties.

The viscosity of the compound (B) is preferably in the range of 5 to 500 cP, more preferably in the range of 20 to 300 cP at 25°C. A compound (B) having excessively low viscosity vaporizes during curing under heat, prohibiting production of sufficiently cured product. If the viscosity is too high, the curing speed may be reduced.

Typical examples of the compounds (B) include compounds having dimethylsiloxane and/or diphenylsiloxane units, in the following structure.

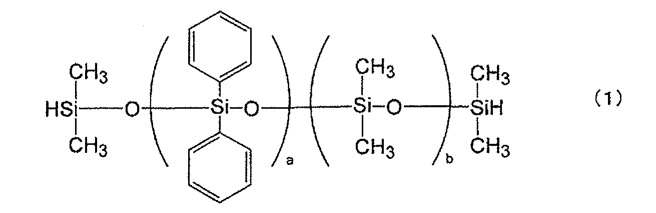

In Formula (1) above, a and b each independently represent 0 (zero)or a positive integer. The values of a and b are not particularly limited, it both of a and b are zero, but each of them is preferably 1 or more, from the point of compatibility with polymer (A), compound (C), and catalyst (D) and dispersion stability. In particular, at least a is preferably 1 or more, (in other words, the diphenylsiloxane unit is essentially contained), to obtain the advantageous effects of the present invention sufficiently. The value of a+b is not particularly limited, but preferably 2 to 50. The production method for the compound (B) is not particularly limited, and, for example, it can be prepared by the method described in JP-A No. 5-32783.

In the present invention, although the detailed mechanism of the cured adhesive agent composition showing favorable adhesive force is unknown, it seems that it is advantageous for expression of adhesive properties to make the cured product suitably crosslinked. It is thus not possible to obtain a cured product showing adhesive properties, because it is not crosslinked favorably when the compound (B) alone is reacted with the polymer (A). It is possible by combined use of the compound (C) to cause favorable crosslinking and to give a cured product superior in adhesive properties.

The compound (C) is a compound having averagely two or more hydrosilyl groups in one molecule. The chemical structure of the compound (C) except the hydrosilyl group is not particularly limited. The number-average molecular weight of the compound (C) is preferably 400 to 4000, more preferably 500 to 2000. It is because excessively low number-average molecular weight makes the compound (C) more volatile during curing under heat, prohibiting production of sufficiently cured product, while excessively high molecular weight leads to decrease in curing speed.

The number of the hydrosilyl groups contained in one molecule of the compound (C) is averagely 2 to 10, preferably 2 to 8. If the compound (C) has three or more hydrosilyl groups, it can crosslink multiple polymer (A) molecules during curing, providing the adhesive layer with a cohesive force favorable as skin adhesive, and suppressing deposition of the adhesive layer on the skin, when it is adhered to and separated from the skin. However, depending on the number of the hydrosilyl group, crosslinking may proceed too densely and the compound (C) may cause deterioration of adhesive properties as skin adhesive, such as skin adhesive force and tackiness. The density of crosslinking exerts influence on the density of the polyether regions in the main chain of the polymer (A) and also on the moisture permeability of the entire adhesive. Thus, the number of the hydrosilyl group in the compound (C) should be selected, as the balance with the adhesive properties is taken into consideration. The compounds (C) may be used alone or in combination of two or more.

Preferably, the compound (C) is favorably compatible with the polymer (A). Compounds (C) favorable from commercial availability of raw materials and also from compatibility with the polymer (A) are, for example, organohydrogensiloxanes modified with organic groups. Typical examples of the organohydrogensiloxanes are the compounds represented by the following Formula (6).

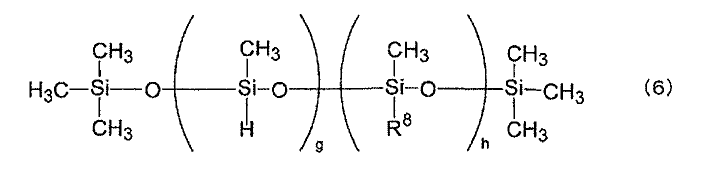

In Formula (6) above, g and h each represent a positive integer, and the value g corresponds to the number of the hydrosilyl groups in molecule. The values of g and h are not particularly limited, if the number of hydrosilyl groups is averagely two or more. The value of g+h is also not particularly limited, but preferably 2 to 50.

R⁸ represents a hydrocarbon group having 2 to 20 carbon atoms in the main chain. The compound of Formula (6) can be obtained by introducing R⁸ into unmodified methylhydrogensilicone by modification. The unmodified methylhydrogensilicone is a compound represented by the following Formula (7)

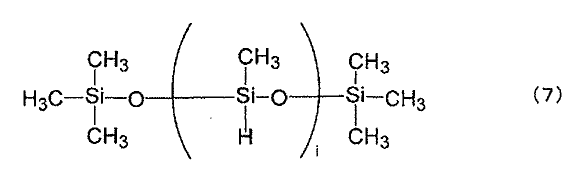

(wherein, i is a positive integer), and used as a raw material for various modified silicones, as described in "Forecast of Silicone Market; Strategies of Makers and Applications", CMC Publishing Co., Ltd (1990.1.31).

Examples of the organic compounds used for introduction of R⁸ include α-olefins, styrene, α-methylstyrene, allyl alkyl ethers, allyl alkyl esters, allyl phenyl ether, allyl phenyl ester, and the like. The number of the hydrosilyl groups in molecule can be adjusted, by the amount of the organic compounds described above added for modification.

The rate in amount of polymer (A) and compounds (B) and (C) in the adhesive to be coated on the base material is expressed by the ratio of the number of the hydrosilyl groups derived from component (B) and (C) to the number of the alkenyl groups derived from component (A).

As described above, use of the compound (B) alone does not results in favorable crosslinking of the polymer (A), thus making it difficult for the composition to express favorable adhesive properties. As for the mechanism of the composition according to the present invention showing favorable skin-adhering properties, it was considered that favorable adhesive properties are expressed by extension of the chain length of the polymer (A) and increase of the molecular weight of the region between crosslinking points in the cured product. Thus in the present invention, favorable adhesive properties are expressed by combined use of particular amounts of compounds (B) and (C).

When the favorable adhesive force is taken into consideration, a ratio of [total amount of hydrosilyl group in component (B)]/[total amount of alkenyl group in component (A)] in the adhesion agent composition is preferably 0.1 to 0.9 ,more preferably 0.3 to 0.9. At a ratio of more than 0.9, it is difficult to obtain a favorable cured product because of the reason described above, while at a ratio of less than 0.1, it is difficult to achieve the adhesive force needed for adhesion to the skin.

Alternatively when the suitable aggregating efficiency and the adhesive force of the cured product are taken into consideration, a ratio of [total amount of hydrosilyl group in component (C)]/[total amount of alkenyl group in component (A)] in the adhesion agent composition is preferably 0.1 to 0.9, more preferably 0.2 to 0.7. A ratio of more than 0.9 is unfavorable, because it leads to excessively dense crosslinking, making it difficult to express adhesive force, while a ratio of 0.1 is also unfavorable, because it leads to excessively coarse crosslinking, raising concerns about deposition of adhesive residues after separation and deterioration in properties at high temperature.

Alternatively when the stability of the cured product is considered, the ratio of the total amount of the alkenyl groups in component (A) to the total amount of the hydrosilyl groups derived from component (B) and (C) is preferably 1.5 or less. The cured product from an adhesion agent composition at a ratio of more than 1.5 is not favorable, because there are concerns that further curing of the cured product may proceed with the reactive hydrosilyl groups present in excess, leading to change in adhesive properties over time.

The hydrosilylation catalyst of component (D) is not particularly limited, and any compound may be used if it accelerates hydrosilylation reaction. Typical examples thereof include chloroplatininic acid, pure platinum, carriers such as alumina, silica and carbon black carrying solid platinum, platinum-vinylsiloxane complexes (such as platinum-1,3-divinyl-1,1,3,3-tetramethyldisiloxane complex and platinum-1,3,5,7-tetravinyl-1,3,5,7-tetramethylcyclotetrasiloxane complex), platinum-olefin complexes (such as Ptx(ViMe2SiOSiMe2Vi)y, Pt[(MeViSiO)4)]z (wherein, x, y, and z each represent a positive integer)) and the like.

In particular, platinum complex catalysts containing no conjugate base of strong acid as ligand are preferable from the point of catalyst activity; platinum-vinylsiloxane complexes are more preferably; and platinum-1,3-divinyl-1,1,3,3-tetramethyldisiloxane complex or platinum-1,3,5,7-tetravinyl-1,3,5,7-tetramethylcyclotetrasiloxane complex are particularly preferable.

The amount of the catalyst (D) is not particularly limited, but preferably 10⁻⁸ to 10⁻¹ mole, more preferably 10⁻⁶ to 10⁻² mole with respect to 1 mole of the total amount of the alkenyl group derived from polymer (A). It is easily possible for example to obtain suitable curing speed and reliable curing efficiency, and to assure needed pot life when the amount of the catalyst (D) is in the range above.

The adhesive agent composition for forming the pressure-sensitive adhesive layer may contain components other than components (A) to (D) above. Examples of the components include tackifiers, storage stabilizers for compounds (B) and (C), and other components.

Examples of the tackifiers include phenol resins, modified phenol resins, terpene phenol resins, xylene phenol resins, cyclopentadiene-phenol resins, xylene resins, petroleum resins, phenol-modified petroleum resins, rosin ester resins, low-molecular weight polystyrene resins, terpene resins, and the like. If the tackifiers are used for improvement in adhesive properties, they may be used alone or in combination of two or more. The amount of the tackifiers, when used, is preferably 10 to 100 wt parts, more preferably 15 to 50 wt parts with respect to 100 wt parts of the total amount of polymer (A) and, compounds (B), and (C). Excessive use of it is unfavorable, as it leads to deterioration of the moisture permeability of adhesive agent.

Examples of the storage stabilizers for compounds (B) and (C) include aliphatic unsaturated bond-containing compounds, organic phosphorus compounds, organic sulfur compounds, nitrogen-containing compounds, tin compounds, organic peroxides, and the like. Typical examples thereof include, but are not limited to, 2-benzothiazolylsulfide, benzothiazole, thiazole, dimethyl acetylenedicarboxylate, diethyl acetylenedicarboxylate, 2,6-di-t-butyl-4-methylphenol, butylhydroxyanisole, vitamin E, 2-(4-morphodinyl dithio)benzothiazole, 3-methyl-1-buten-3-ol, acetylenic unsaturated group-containing organosiloxanes, acetylenealcohol, 3-methyl-1-butyn-3-ol, 2-methyl-3-butyn-2-ol, diallyl fumarate, diallyl maleate, diethyl fumarate, diethyl maleate, dimethyl maleate, 2-pentenenitrile, 2,3-dichloropropene, and the like.

The storage stabilizer suppresses conversion of the hydrosilyl groups (Si-H groups) in compounds (B) and (C) to Si-OH groups (due to storage for an extended period or contamination of moisture) and elongates the pot life of the paint. The amount of the storage stabilizer blended is preferably 10⁻⁶ to 10⁻¹ mole with respect to 1 mole of the total amount of the hydrosilyl groups derived from compounds (B) and (C) contained in the adhesive agent composition.

The adhesive agent composition for preparation of the pressure-sensitive adhesive layer may contain at least one hydrophilic polymer in the form selected from particle and fiber for improvement of the water resistance, perspiration resistance, water absorption, and others of the pressure-sensitive adhesive layer. Typical examples thereof include hydrophilic polymers such as acrylic acid-modified starch, polyacrylic acid, sodium polyacrylate, carboxymethylcellulose (CMC), sodium carboxymethylcellulose (CMCNa), polyvinylalcohol (PVA), polyvinylpyrrolidone (PVP), methylvinylether maleic anhydride copolymers, sodium alginate, alginic acid propylene glycol ester, pectin, xanthan gum, locust bean gum, guar gum, arabinogalactan, sodium hyaluronate and the like. These hydrophilic polymers may be used, as needed, in combination of two or more.

In addition, plasticizers, softeners, fillers, pigments, surfactants, ultraviolet absorbents, antioxidants, antibacterial agents, pharmaceutical components and others may also be blended. No organic solvent is preferably used then, but use of organic solvents is not denied.

The method of forming a pressure-sensitive adhesive layer on the support is not particularly limited, and examples thereof include a method of applying the adhesive agent composition on one face of the support and curing it under the condition described above and a method of applying the adhesive agent composition on a release agent-coated sheet (release sheet) and bonding it after curing to a cured support. Various release agents such as silicone-, olefin- and fluorine-based release agents are known as the release agents and can be used as properly selected. In particular, non-solvent addition-curing silicone-based release agents are preferable from the points of cost and release characteristics.

The viscosity of the adhesive agent composition when applied is preferably 10 to 1000 Pa·s. The viscosity can be regulated by adjustment of the amount ratio of the components (A) to (D) and the kind and amount of the storage stabilizer for compounds (B) compound (C) describe above.

An adhesion-improving agent for improvement of adhesiveness to various supports may be applied to the support, as needed, before the adhesive agent composition according to the present invention is laminated. Examples of the adhesion-improving agents include various silane-coupling agents, epoxy resins, and the like. In particular, silane-coupling agents having a functional group such as epoxy, methacryloyl or vinyl are favorably used, as they exert smaller adverse influence on curing efficiency and are yet effective for expression of adhesiveness. However, the usable silane-coupling agents are not limited thereto. The thickness of the pressure-sensitive adhesive layer is not particularly limited and may be, for example, 10 to 5000 µm.

The pressure-sensitive adhesive sheet to be stuck to the skin according to the present invention means generally a sheet adhered to the skin for treatment and prevention of disease and injury, diagnosis of health condition or fixation of a medical device on the skin surface. The adhesive sheet may contain or may not contain a physiologically active substance. Typical examples thereof include, but are not limited to, wound dressings, adhesive bandages, skin-protecting adhesive sheets, wound-protecting materials, wound-preventing materials, film dressing materials for wound treatment, surgical tapes, athletic tapes, percutaneously absorbing agents, and the like.

### Examples

Hereinafter, the present invention will be described more specifically with Examples, but it should be understood that the present invention is not limited thereto and various modification thereof is possible within the technical scope of the present invention. Compounds A, B, and others in the following description are as follows:
A: Polyoxyalkylene polymer having at least one alkenyl group at the terminal
A-1: Polyoxyalkylene having allyl groups at the terminals (see Preparative Example below, containing 0.12 mmol/g alkenyl group)
B: Compound having hydrosilyl groups at both terminals
B-1: Silicone oil (containing 1.16 mmol/g hydrosilyl group)
B-2: Silicone oil (containing 1.69 mmol/g hydrosilyl group)
B-3: Silicone oil (containing 0.76 mmol/g hydrosilyl group)
C: Compound having averagely two or more hydrosilyl groups in molecule
C-1: Silicone oil (containing 3.2 mmol/g hydrosilyl group)
C-2: Silicone oil (containing 4.2 mmol/g hydrosilyl group)
D: Hydrosilylation catalyst
D-1: Platinum-1,3-divinyl-1,1,3,3-tetramethyldisiloxane complex (3 wt % platinum isopropanol solution).

### (Comparative Example 1) Synthesis of polymer (A-1)

An oxypropylene polymer glycol having a number-average molecular weight of 3000 was prepared by a polymerization method using a caustic alkali. Propylene oxide was polymerized according to the method of Preparative Example 1 in JP-A No. 5-117521 by using the oxypropylene polymer glycol as initiator and a mixed metal cyanide complex catalyst (zinc hexacyanocobaltate), to give a polymer having a number-average molecular weight of 28000. The terminals of the polymer were bonded to allyl groups, by using allyl chloride and 28% methanol solution of sodium methylate, and the reaction product was demineralized and purified to give a polyoxyalkylene polymer (polymer (A-1)) having approximately two allylated terminals in the molecule. The amount of the allyl terminal group of the obtained polyoxyalkylene polymer was 0.12 mmol/g.

### (Comparative Example 2) Synthesis of compound (B-1)

A compound (B-1) containing dimethylsiloxane and diphenylsiloxane units and having hydrosilyl groups at both terminals was prepared by the method described in JP-A No. 5-32783. The hydrosilyl group content of this compound was 1.16 mmol/g, and the refractive index thereof was 1.482 and the viscosity 54 cP at 25°C.

### (Comparative Example 3) Synthesis of compound (B-2)

A compound (B-2) containing dimethylsiloxane and diphenylsiloxane units and having hydrosilyl groups at both terminals was prepared by the method described in JP-A No. 5-32783. The hydrosilyl group content of this compound was 1.69 mmol/g, and the refractive index thereof was 1.480 and the viscosity 32 cP at 25°C.

### (Comparative Example 4) Synthesis of compound (B-3)

A compound (B-3) containing dimethylsiloxane and diphenylsiloxane units and having hydrosilyl groups at both terminals was prepared by the method described in JP-A No. 5-32783. The hydrosilyl group content of this compound was 0.76mmol/g, and the refractive index thereof was 1.483 and the viscosity 81 cP at 25°C.

### (Comparative Example 5) Synthesis of compound (C-1)

0.5 equivalente amount of α-Methylstyrene with respect to a total amount of hydrosilyl group was added to the methylhydrogensilicone represented by Formula (7) above (wherein, i is an average of 5) in the presence of a platinum catalyst, to give a compound having averagely 2.5 hydrosilyl groups in the molecule (compound (C-1)). The hydrosilyl group content of this compound was 3.2 mmol/g.

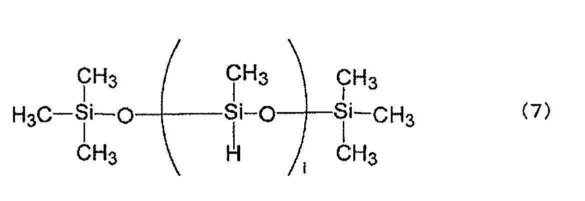

### (Comparative Example 6) Synthesis of compound (C-2)

0.5 equivalente amount of α-Methylstyrene with respect to a total amount of hydrosilyl group was added to the methylhydrogensilicone represented by Formula (7) above (wherein, i is an average of 10) in the presence of a platinum catalyst, to give a compound having averagely 5 hydrosilyl groups in the molecule (compound (C-2)). The hydrosilyl group content of this compound was 4.2 mmol/g.

### (Examples 1 to 7 and Comparative Examples 1 to 2)

A compound B (B-1, B-2 or B-3) and a compound C (C-1 or C-2) were mixed to the polymer A (A-1) in the amounts shown in the Table 1, and additionally, 0.1 wt part of a hydrosilylation catalyst D (D-1) and 0.03 wt part of 2-methyl-3-butyn-2-ol were added to and mixed with it sufficiently, to give an adhesive agent composition. The adhesive agent composition was applied on the release-finished surface of a release liner treated with silicone to a post-curing thickness of 50 µm and cured thereon at 130°C for 3 minutes, to form a pressure-sensitive adhesive layer.

### (Adhesive force)

The pressure-sensitive adhesive layer obtained in the Example above was applied on a support polyester film (Toray Industries, Inc., Lumirror, film thickness: 25 µm) under the condition of an application speed of 2 m/min with a rubber roller having a weight of 2 kg, to give an adhesive sheet. The sheet was cut into strips of 25 mm in width, and a strip was adhered to a SUS 304 plate with a rubber roller having a weight of 2 kg under the condition of a speed of 2 m/min and left for 1 hour. The stress applied to each tape when it was peeled off from the SUS 304 plate to an angle of 180°at a speed of 300 mm/min was used as the measured adhesive force of the tape. Results are summarized in Table 1.

**[Table1]**

| | A-1 | B-1 | B-2 | B-3 | C-1 | C-2 | SiH group/allyl group | Adhesive force |
|---|---|---|---|---|---|---|---|---|
| | (g) | (g) | (g) | (g) | (g) | (g) | (molar ratio) | (N/25mm) |
| Example 1 | 100 | 4.0 | | | 2.0 | | 0.98 | 3.75 |
| Example 2 | 100 | 5.0 | | | 1.6 | | 0.96 | 4.31 |
| Example 3 | 100 | | 3.4 | | 1.7 | | 0.98 | 3.83 |
| Example 4 | 100 | | | 6.2 | 2.0 | | 0.98 | 3.81 |
| Example 5 | 100 | 8.0 | | | | 1.0 | 1.14 | 3.50 |
| Example 6 | 100 | 4.6 | | | | 0.7 | 0.70 | 3.50 |
| Example 7 | 100 | 4.6 | | | | 1.0 | 0.81 | 2.88 |
| Comparative Example 1 | 100 | | | | 2.2 | | 0.64 | 2.43 |
| Comparative Example 2 | 100 | | | | 3.6 | | 1.05 | 0.28 |

## Claims

1. A pressure-sensitive adhesive sheet to be stuck to the skin having a support and a pressure-sensitive adhesive layer formed thereon, **characterized in that**, the pressure-sensitive adhesive layer is prepared by curing an adhesive agent composition containing (A) a polyoxyalkylene polymer having at least one alkenyl group in one molecule, (B) a compound having hydrosilyl groups at both ends, (C) a compound having two or more hydrosilyl groups on average in one molecule, and (D) a hydrosilylation catalyst.

2. The pressure-sensitive adhesive sheet to be stuck to the skin according to Claim 1, wherein the compound (B) is a compound represented by the following Formula (1):

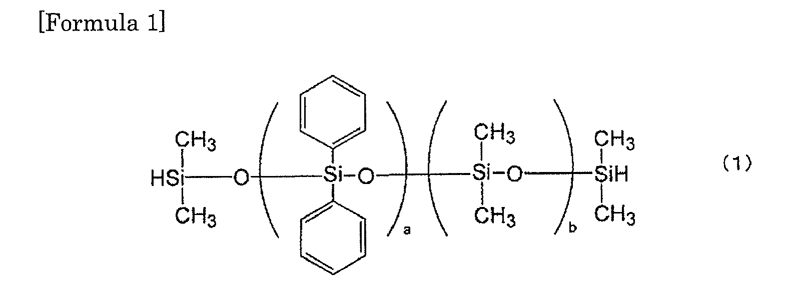

3. The pressure-sensitive adhesive sheet to be stuck to the skin according to Claim 1 or 2, wherein the compound (B) has a number-average molecular weight of 300 to 4000.

4. The pressure-sensitive adhesive sheet to be stuck to the skin according to any one of Claims 1 to 3, wherein the compound (B) has a refractive index of 1.45 to 1.55 at 25°C.

5. The pressure-sensitive adhesive sheet to be stuck to the skin according to any one of Claims 1 to 4, wherein the compound (B) has a viscosity of 5 to 500 cP at 25°C.

6. The pressure-sensitive adhesive sheet to be stuck to the skin according to any one of Claims 1 to 5, wherein the polyoxyalkylene polymer (A) has a number-average molecular weight of 3000 to 50000.

7. The pressure-sensitive adhesive sheet to be stuck to the skin according to any one of Claims 1 to 6, wherein the main chain of the polyoxyalkylene polymer (A) is polyoxypropylene.

8. The pressure-sensitive adhesive sheet to be stuck to the skin according to any one of Claims 1 to 7, wherein the adhesive layer is prepared by curing an adhesive agent composition having a ratio of [total amount of hydrosilyl group in component (B)]/[total amount of alkenyl group in component (A)] in the range of 0.1 to 0.9.
